# EUROPEAN PATENT SPECIFICATION

(11) **EP 0 490 517 B1**
(45) Date of publication and mention of the grant of the patent: **17.01.1996**
(21) Application number: 91310889.0
(22) Date of filing: 26.11.1991
(51) Int. Cl.: A61B 17/34

(54) **Intraosseous needle**
Intraossale Nadel
Aiguille intra-osseuse

(30) Priority: 13.12.1990 US 627020
(43) Date of publication of application: 17.06.1992
(73) Proprietor: Cook Incorporated, Bloomington Indiana 47402 (US); UNIVERSITY OF FLORIDA RESEARCH FOUNDATION, INC., Gainesville, FL 32611 (US)
(72) Inventor: Miller, Gary J., Gainesville, Florida 32601 (US); DeBruyne, Michael P., Bloomington, Indiana 47408 (US); Molitor, Lisa, Gainesville, Florida 32601 (US); Melker, Richard J., Gainesville, Florida 32605 (US); Gearen, Peter F., Gainesville, Florida 32601 (US)
(74) Representative: Bannerman, David Gardner

(56) References cited:
- EP-A- 0 102 605
- WO-A-90/04364
- US-A- 3 750 667

## Description

The present invention relates to infusion needles, and more particularly, to an intraosseous infusion needle having a tip adapted to bore directly into the patient's bone.

In a variety of medical emergencies, the patient's life may hinge upon the ability of the physician or medical attendant to administer a particular fluid into the patient's bloodstream. In emergency situations such as on the battlefield, at traffic accident scenes or in the emergency room, the patient is often in shock, has low blood pressure, is bleeding profusely and may be thrashing about. Under such circumstances, finding and gaining access to a suitable blood vessel can be all but impossible, the resulting delay in administering drugs to the patient possibly being fatal. In the case of children or infants in any emergency, even the largest veins are so small that they may not be located. Even if located, an infant's largest available vein may be so small that stable infusion access may not be possible.

One alternative to venous access, recently reintroduced, is the intraosseous route. The medullary cavity of the long bones is composed of a rich spongy network of venous sinusoids which drain into a central venous canal. Blood then exits the venous canal by nutrient or emissary veins into the circulation. Fluids or drugs injected into the medullary area usually diffuse only a few centimeters, enter the bloodstream and reach the heart--all in only about 10 seconds from injection into the medullary cavity. It is important that any devices which provide intraosseous access have a lumen of a size adequate to allow for the infusion of relatively large volumes of fluid over a short period of time. Current intraosseous infusion procedures (meaning before the improvement described herein) utilize a hollow needle having a beveled tip and a trocar or stylet. With the stylet telescopically positioned within and extending partially out the bevelled end of the needle, the needle and trocar assembly is forceably and perpendicularly advanced against and through the desired bone until the cortex has been punctured and the needle and trocar tip has advanced into the medullary space. The trocar is then withdrawn, leaving the open end of the needle directly in the rich vascular network. Various complications, however, have made intraosseous infusion a less than ideal option. Although the needle and trocar assembly have a sharp, pointed tip, the medullary cavity may not be able to be penetrated under normal pressure. Too much force in trying to puncture the bone sometimes results in a bent needle, a broken needle, splintering of the bone, sliding off the bone and puncturing adjacent tissue or, more commonly, the needle is accidentally forced through the opposite side of the bone. Even if the needle is properly inserted into the medullary cavity, movements by the patient can easily dislodge the needle or cause it to be moved so that the end opening is occluded. These complications commonly arise in cases involving intraosseous infusion of infants and children. Additionally, fluid may leak from the puncture site into surrounding tissues. For persons older than six, the bones are too hard to successfully perform intraosseous infusion utilizing current procedures without realizing an extremely high incidence of the above complications. The current procedure has, therefore, typically been limited to children less than six years old and only after several attempts have been made to achieve venous infusion.

WO-A-90/04364 describes an intraosseous needle with generally conical boring means having a pointed tip and at least two cutting edges.

What is needed is an intraosseous infusion device which decreases the incidence and severity of the above described complications, which is easier to insert, which is more stable once inserted, prevents leakage of infused fluids, and, most importantly, which can be used in subjects of all ages.

Generally speaking, there is provided an intraosseous needle which allows for precise control and placement of the needle during intraosseous infusion procedures.

According to the invention there is provided an intraosseous needle comprising:
a threaded shaft having first and second opposing ends and a passageway extending from said first end towards said second end, said second end having integrally formed thereon a boring means for initiating penetration of said shaft into bone,
wherein said shaft defines an axis and includes a first side port proximal to said second end and axially aligned with one of said three cutting edges, said first side port being in communication with said passageway; and
said threads define a helical ridgeline,
characterised in that said boring means is a three-sided pyramid which defines three cutting edges that intersect in a pointed tip, said side port is axially aligned with one of said three cutting edges, and in that said helical ridgeline coincides at a point with one of said three cutting edges, said one of said cutting edges terminating at said point.

Preferably, said needle includes a second side port situated at an angle of approximately 270° from said first side port about said axis.

Preferably, the first side port is located between two adjacent said threads that are uninterrupted by said three sided pyramid.

Preferably, said cutting edges are orientated to enable cutting into bone if said needle is turned about said axis in either a clockwise direction or a counterclockwise direction.

In a preferred intraosseous needle according to the invention, the shaft has a plurality of threads having a thread angle of approximately 45° defining said helical ridgeline, wherein said helical ridgeline coincides at a point with a first one of said three cutting edges to initiate engagement of said threads with the bone, wherein said first side port is axially aligned with a second one of said three cutting edges of said three-sided pyramid and located between the first two adjacent said threads that are uninterrupted by said second one of said three cutting edges, and wherein said second side port is located between two adjacent said threads.

Embodiments of the invention will now be described by way of example and with reference to the accompanying drawings, in which:
FIG. 1 is a plan view of an intraosseous needle in accordance with one embodiment of the present invention.
FIG. 2 is a fragmented plan view of the needle of FIG. 1 which has been rotated 90° about its axis.
FIG. 3 is a plan view, partly in section, of a handle and intraosseous needle of the present invention.
FIG. 4 is a front view of the handle of FIG. 3.
FIG. 5 is a front end view of the intraosseous needle of FIG. 1.
FIG. 6 is a fragmented plan view of an intraosseous needle in accordance with the preferred embodiment of the present invention.
FIG. 7 is a front end view of the intraosseous needle of FIG. 6 showing the angular relationships between side ports and cutting edges.
FIG. 8 is a plan view of the intraosseous needle of FIG 6 rotated about its axis to show the cutting edge which coincides at a point with the ridgeline defined by the threads.

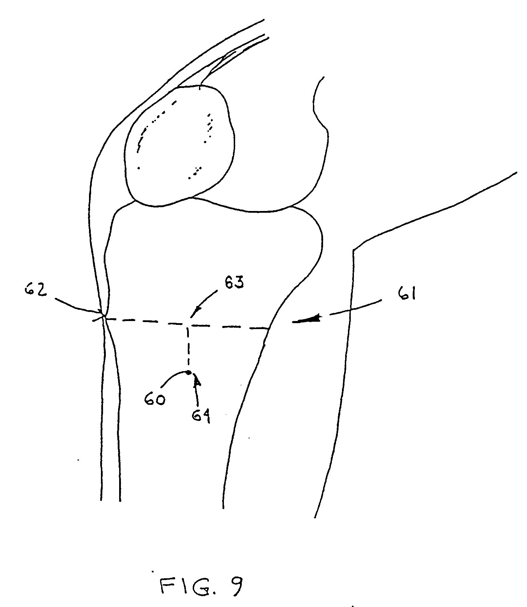

FIG. 9 is a diagrammatic side view of the proximal tibia showing the preferred access site of the present invention.

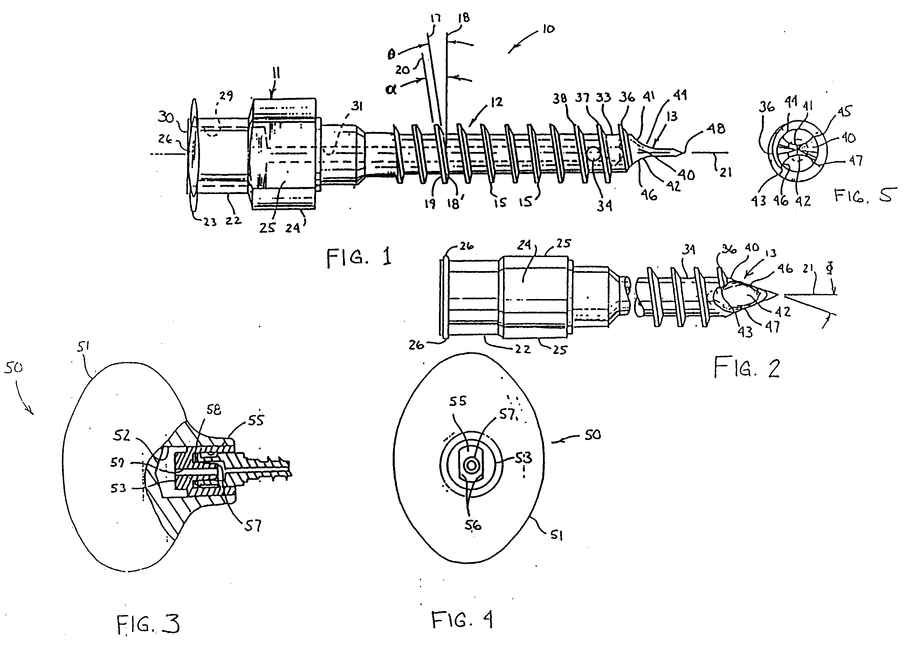

Referring now to FIGS. 1 and 2 there is shown an intraosseous needle 10 in accordance with one embodiment of the present invention. Needle 10 includes a hub 11, a threaded shaft 12 and boring means. In this embodiment, the boring means is a fluted pencil point tip 13. Behind the boring means, there are 0.150-16 buttress threads 15 on shaft 12 having a major diameter of 0.38cm (0.150") and a minor diameter of 0.27cm (0.110"), both to a tolerance of 0.13cm (0.005"). Recall that the threads are shaped to ensure that the needle is stable and prevents leakage once inserted. The lead angle or helix angle θ is defined as the angle formed by a plane (indicated at 17) drawn tangent to the pitch helix and a plane (indicated at 18) normal to the axis 21 of threaded shaft 12. The leading and trailing thread surfaces are indicated at 18' and 19, respectively. The trailing thread angle α is defined here as that angle formed by a plane (indicated as 20) drawn tangent to trailing thread surface 19 and plane 18 normal to axis 21. Trailing thread angle α in the preferred embodiment is equal to the helix angle θ. That is, plane 17 is parallel to plane 20. As shown in the embodiment of Fig. 1, trailing thread angle alpha is approximately 8°, while leading surface 18' forms an angle with normal plane 18 of about 30°. That is, leading and trailing surfaces 18' and 19 are not parallel.

Hub 11, located at the rearward or trailing end of threaded shaft 12, forms the female end for connection to a conventional Luer-type fitting and includes a generally cylindrical portion 22 and an annular flange portion 23. A generally cylindrical section 24 is located between cylindrical portion 22 and threaded shaft 12. The diameters of flange portion 23 and cylindrical section 24 are approximately equal and both are greater than the diameter of cylindrical portion 22. Cylindrical section 24 has a pair of diametrically opposed and mutually parallel flat faces 25. Flange portion 23 likewise has a pair of diametrically opposed and mutually parallel flat faces 26 which are coplanar with corresponding flat faces 25. A large diameter bore 29 with a standard Luer taper is defined in hub 11 and extends from end 30 through cylindrical portion 22 and partially through cylindrical section 24. Bore 29 receives the male portion of the Luer-type fitting. A smaller diameter axial passageway 31 is in communication with bore 29 and extends from bore 29 forwardly through nearly the entire length of threaded shaft 12. A pair of side ports 33 and 34 extend radially outwardly from axial passageway 31 near tip 13. Side ports 33 and 34 are located 90° apart. Side port 33 opens outwardly in the valley between the leading or first full thread 36 and the second thread 37. Side port 34 opens outwardly in the valley between the second thread 37 and the third thread 38.

Fluted pencil point tip 13 is substantially conical with the conical outer surface 40 forming an angle Φ with axis 21 of approximately 20°. A pair of diametrically opposed flutes 41 and 42 are milled into the end of tip 13 using a ball end mill. The end mill used to cut flutes 41 and 42 is aligned to rotate about an axis which is parallel with axis 21 during the milling process. The foremost end 43 of leading thread 36 is interrupted by the milling process such that leading thread 36 terminates into one of the flutes 42. The milling process thus forms sharp boring edges 44 and 45 between flute 41 and conical surface 40 and sharp boring edges 46 and 47 between flute 42 and conical surface 40. The border between leading thread 36 and flute 42 likewise forms a sharp cutting edge at 43. As shown in FIG. 1, flute 42 is machined further rearwardly than flute 41. In the present embodiment, the complete axial length of flute 41 measured from tip 48 is 0.35cm (0.14 inches) while the complete axial length of flute 42 measured from tip 48 is 0.40cm (0.16 inches).

A complete intraosseous needle assembly includes, along with intraosseous needle 10, a corresponding gripping means or gripping element which is handle 50 (FIGS. 3 and 4). Handle 50 comprises a plastic ball knob 51 having an axial bore 52. An insert 53 for gripping needle 10 is sized to be tightly received within bore 52. Insert 53 is fixed within bore 52 by appropriate means such as by gluing. Insert 53 is adapted to couple with needle 10 and has a central opening 55 which is generally cylindrical with opposing planar faces 56. Opening 55 is sized to receive the complimentary shape of hub 11 with its cylindrical section 24 and opposing flat faces 25. Insert 53 further includes stub 57 which extends forwardly into opening 55. As hub 11 of needle 10 is received within opening 55, stub 57 enters bore 29 of hub 11. Stub 57 is stepped slightly forwardly such that its largest diameter, at its base 58, is the same as or just slightly larger than the inner diameter of bore 29. As stub 57 advances into hole 29, the larger diameter at base 58 of stub 57 wedges within bore 29 forming a snug fit between needle 10 and handle 50. Hub 11, bore 29, opening 55 and stub 57 are sized to create a mutually snug connection sufficient to cause hub 11 to remain firmly lodged within handle 50 but to be removed under a moderate manually applied tensile force.

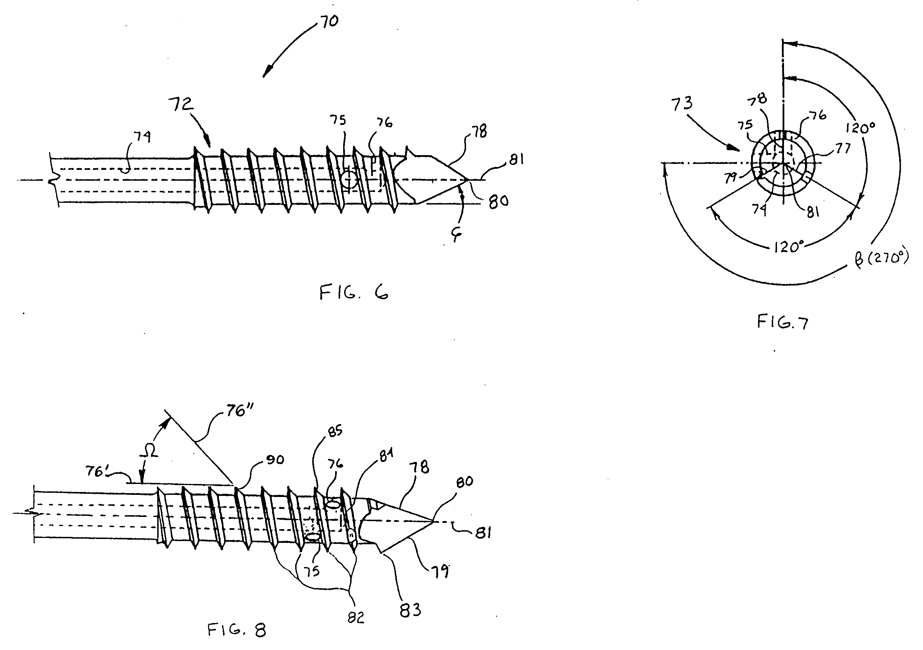

Referring now to FIGS. 6, 7 and 8, there is shown an intraosseous needle 70 in accordance with a preferred embodiment of the present invention. Needle 70 includes a hub, not shown but identical to hub 11 described earlier, a threaded shaft 72 and a boring end 73. Like the embodiment described earlier, shaft 72 includes an axial passageway 74 in communication with side ports 75 and 76. Also, the thread size is identical to the embodiment described earlier, which ensures the stability of the needle once implanted and the patency of the side ports (described infra). Like the embodiment described above, the thread angle Ω is approximately 45°. The thread angle Ω is the angle measured between a line 76' parallel to axis 81 and a line 76'' which extends through the axis 81 and is coextensive with the face 90 of the thread. The boring end 73 is formed in the shape of a regular three-sided pyramid wherein the three cutting edges 77, 78 and 79 culminate in pointed tip 80. Cutting edges 77, 78 and 79 are 120° apart about axis 81, which is defined by the needle. Boring end 73 can be formed by grinding or any other suitable means which leaves cutting edges 77, 78 and 79 relatively sharp. The grind angle ç, which in this case is approximately 24°, defines the slope of each side of the pyramid. The boring end 73 is precision ground so that cutting edge 79 coincides with helical thread ridgeline 82 at point 83. This arrangement couples boring end 73 to threaded shaft 72, allowing the threads to engage the bone when the boring is completed.

Also of significance is the location of side ports 75 and 76 with respect to both the threads of shaft 72 and the cutting edges of boring end 73. Side ports 75 and 76 must be sized and situated to avoid the risk that they might become clogged with tissue during insertion of needle 70 into bone in order to ensure that potentially life-saving fluids can be passed through the needle to the desired location within the bone. With this in mind, side port 76 is axially aligned with cutting edge 78 and is located in the valley between the first two successive threads 84 and 85 which are uninterupted by the surfaces and edges forming boring end 73. Side port 75 is likewise situated between two adjacent threads but is angularly displaced about axis 81 from side port 76. This angle β is approximately 270°.

The intraosseous needle assembly is used as follows:

An intraosseous needle 10 or 70 is firmly secured to a handle 50. The preferred site is marked and an incision is made in the skin down to the bone. The preferred site 64 is found by first identifying the tibial tuberosity 62 on the anterior surface of the proximal tibia. An imaginery line is drawn from the tibial tuberosity to the median edge of the tibia 61. This line is equally divided 63 and the site of insertion 64 is perpendicular and distal to 63. The preferred site 64 increases in distance from 63 with increasing age. In the newborn or infant this distance may be as short as 0.3 - 0.5 cm and increases to approximately 2.5 cm by 6 years of age. Insertion at the level of the tibial tuberosity or distally, avoids insertion of the needle into the growth plate of the tibia. The distal medial tibia is also an excellent site.

With handle 50 firmly in the palm of the operator's hand, the needle is selectively directed toward the desired access site 63 and contact with the bone is made. A back and forth twisting motion with slight pressure causes the four cutting edges 44-47, or the three cutting edges 77-79, to cut into the bone. This enables flutes 41 and 42, as well as cutting edges 77-79, to penetrate to the threaded section of the needle. During the twisting motion, flutes 41 and 42, as well as the pyramid surfaces in the preferred embodiment, carry bone fragments out of the hole. Once the lead thread 36 of needle 10 reaches the hole 60, no further pressure is required. In the case of needle 70, the back and forth twisting motion is continued until point 83 engages the bone. After engaging the threads, the operator simply screws the needle clockwise into the marrow to the desired depth. Obviously the size of the patient will determine how far to screw the needle in. As the needle is rotatably advanced, the design of the threads directs the marrow out and away from the side ports. A fluid injected through needle 10, or 70, may then exit through side ports 33 and 34, or 75 and 76, unobstructed by marrow or other tissue which otherwise clogs conventional intraosseous needles.

With needle 10 or 70 in the desired position, handle 50 may be detached from the needle by slight, manually applied, tensile pressure therebetween. The appropriate drug administering mechanism such as a syringe or I.V. tubing may then be secured via the Luer-type fitting. After the patient has stabilized, venous access may be achieved and needle 10 or 70 may be removed by detaching the I.V. tubing or syringe from the needle and by re-securing handle 50 thereto. The needle 10 or 70 may then be backed out by turning the needle counterclockwise.

## Claims

1. An intraosseous needle (70) comprising:
a threaded shaft (72) having first and second opposing ends and a passageway (74) extending from said first end towards said second end, said second end having integrally formed thereon a boring means (73) for initiating penetration of said shaft into bone,
wherein said shaft defines an axis and includes a first side port (76) proximal to said second end, said first side port being in communication with said passageway; and
said threads define a helical ridgeline (82), characterised in that said boring means is a three-sided pyramid which defines three cutting edges (77, 78, 79) that intersect in a pointed tip (80), said side port (76) is axially aligned with one of said three cutting edges, and in that said helical ridgeline coincides at a point with one (79) of said three cutting edges, said one (79) of said cutting edges terminating at said point.

2. The intraosseous needle of claim 1, wherein said needle includes a second side port (75) situated at an angle of approximately 270° from said first side port about said axis.

3. The intraosseous needle of claim 2 wherein said first side port (76) is located between two adjacent said threads that are uninterrupted by said three sided pyramid.

4. The intraosseous needle of claim 3 wherein said cutting edges are orientated to enable cutting into bone if said needle is turned about said axis in either a clockwise direction or a counterclockwise direction.

5. An intraosseous needle according to claim 2 wherein the shaft (72) has a plurality of threads (84, 85) having a thread angle (Ω) of approximately 45° defining said helical ridgeline (82), wherein said helical ridgeline (82) coincides at said point with a first one (79) of said three cutting edges to initiate engagement of said threads with the bone, wherein said first side port (76) is axially aligned with a second (78) one of said three cutting edges of said three-sided pyramid and located between the first two adjacent said threads that are uninterrupted by said second one of said three cutting edges, and wherein said second side port (75) is located between two adjacent said threads.

## Patentansprüche

1. Intraossäre Nadel (70) mit einem Gewindeschaft (72) mit ersten und zweiten gegenüberliegenden Enden und einem Durchgang (74), der sich von dem ersten Ende zum zweiten Ende erstreckt, wobei das zweite Ende integral angeformte Bohrmittel (73) zur Einleitung des Eindringens des Schaftes in den Knochen aufweist, wobei der Schaft eine Achse definiert und eine erste Seitenöffnung (76) in der Nähe des zweiten Endes aufweist, wobei die, erste Seitenöffnung in Verbindung mit dem Durchgang steht, und wobei das Gewinde eine schraubenförmige Gratlinie 82 definiert, dadurch gekennzeichnet daß die Bohrmittel eine dreiseitige Pyramide sind, die drei Schneidkanten (77,78,79) definiert, die sich in einer Spitze (80) überschneiden, wobei die Seitenöffnung (76) axial mit einer der drei Schneidkanten ausgerichtet ist, und wobei die schraubenförmige Gratlinie in einem Punkt mit einer (79) der drei Schneidkanten zusammenfällt, wobei die eine (79) der Schneidkanten an diesem Punkt endet.

2. Intraossäre Nadel nach Anspruch 1, wobei die Nadel eine zweite Seitenöffnung (75) aufweist, die in einem Winkel von etwa 270° von der ersten Seitenöffnung über der Achse angeordnet ist.

3. Intraossäre Nadel nach Anspruch 2, wobei die erste Seitenöffnung (76) zwischen zwei benachbarten Windungen angeordnet ist, die durch die dreiseitige Pyramide nicht unterbrochen sind.

4. Intraossäre Nadel nach Anspruch 3, wobei die Schneidkanten derart orientiert sind, daß sie in den Knochen schneiden, wenn die Nadel um die Achse im Uhrzeigersinn oder entgegen Uhrzeigersinn gedreht wird.

5. Intraossäre Nadel nach Anspruch 2, wobei der Schaft (72) eine Vielzahl von Windungen (84,85) aufweist, die einen Gewindewinkel (Ω) von etwa 45° aufweist, wodurch die schraubenförmige Gratlinie (82) definiert ist, wobei diese an dem genannten Punkt mit einer ersten (79) der drei Schneidkanten zusammenfällt, um das Eindringen des Gewindes in den Knochen zu initiiaren, wobei die erste Seitenöffnung ( 76) axial mit einer zweiten (78) der drei Schneidkanten der dreiseitigen Pyramide ausgerichtet und zwischen den ersten beiden benachbarten Windungen angeordnet ist, die durch die zweite der drei Schneidkanten nicht unterbrochen ist, und wobei die zweite Seitenöffnung (75) zwischen zwei benachbarten Windungen angeordnet ist.

## Revendications

1. Aiguille intra-osseuse (70) comprenant une tige filetée (72) comportant une première et une seconde extrémités opposées ainsi qu'un passage (74) allant de la première extrémité à la seconde extrémité, cette seconde extrémité comportant des moyens de perçage (73) formés d'un seul tenant sur celle-ci pour déclencher la pénétration de la tige dans un os, aiguille dans laquelle la tige définit un axe et comprend un premier orifice latéral (76) proche de la seconde extrémité, ce premier orifice latéral étant en communication avec le passage, et le filetage définissant une ligne d'arête hélicoïdale (82), caractérisée en ce que les moyens de perçage sont constitués par une pyramide à trois côtés définissant trois bords de coupe (77, 78, 79) qui se coupent en formant un bout pointu (80), l'orifice latéral (76) étant aligné axialement avec l'un des trois bords de coupe, et en ce que la ligne d'arête hélicoïdale coïncide en un point avec l'un (79) des trois bords de coupe, ce bord de coupe (79) se terminant à l'endroit de ce point.

2. Aiguille intra-osseuse selon la revendication 1, caractérisée en ce que cette aiguille comprend un second orifice latéral (75) situé à un angle d'approximativement 270° par rapport au premier orifice latéral, autour de l'axe.

3. Aiguille intra-osseuse selon la revendication 2, caractérisée en ce que le premier orifice latéral (76) est placé entre deux, adjacents, des filets qui ne sont pas interrompus par la pyramide à trois côtés.

4. Aiguille intra-osseuse selon la revendication 3, caractérisée en ce que les bords de coupe sont orientés pour permettre une coupe dans un os si l'on fait tourner l'aiguille autour de l'axe soit dans le sens des aiguilles d'une montre soit dans le sens inverse des aiguilles d'une montre.

5. Aiguille intra-osseuse selon la revendication 2, caractérisée en ce que la tige (72) comporte une pluralité de filets (84, 85) ayant un angle de filet d'approximativement 45° définissant la ligne d'arête hélicoïdale (82), en ce que cette ligne d'arête hélicoïdale (82) coïncide, à l'endroit du point ci-dessus, avec un premier (79) des trois bords de coupe pour amorcer l'engagement des filets dans l'os, en ce que le premier orifice latéral (76) est aligné axialement avec un second (78) des trois bords de coupe de la pyramide à trois côtés et se place entre les deux premiers, adjacents, des filets qui ne sont pas interrompus par le second des trois bords de coupe, et en ce que le second orifice latéral (75) est placé entre deux, adjacents, des filets.
